# EUROPEAN PATENT SPECIFICATION

(11) **EP 3 934 535 B1**
(45) Date of publication and mention of the grant of the patent: **18.02.2026**
(21) Application number: 20767445.8
(22) Date of filing: 09.03.2020
(51) Int. Cl.: A61B 5/145, A61F 2/02, A61N 1/18

(54) **IMPLANT FOR CONTINUOUS PATIENT MONITORING**
IMPLANTAT ZUR KONTINUIERLICHEN PATIENTENÜBERWACHUNG
IMPLANT POUR SURVEILLANCE CONTINUE

(30) Priority: 07.03.2019 US 201962815159 P
(43) Date of publication of application: 12.01.2022
(73) Proprietor: PROCEPT BioRobotics Corporation, San Jose, CA 95134 (US)
(72) Inventor: ALJURI, Nikolai, Hillsborough, California 94010 (US); MANTRI, Surag, East Palo Alto, California 94303 (US); STAID, Kevin, Lowell, Massachusetts 01852 (US)
(74) Representative: WBH Wachenhausen Patentanwälte PartG mbB
(86) International application number: PCT/US2020/021710
(87) International publication number: WO 2020/181281

(56) References cited:
- WO-A1-2009/036256
- WO-A1-2015/200723
- WO-A2-2009/151876
- WO-A2-2010/067360
- US-A- 6 053 873
- US-A1- 2005 113 894
- US-A1- 2010 261 983
- US-A1- 2015 141 767
- US-A1- 2017 231 738
- US-A1- 2018 042 549
- US-A1- 2018 238 856

## Description

### BACKGROUND

Prior approaches to monitoring patients can be less than ideal. For example, with urological procedures, tissue can grow subsequent to a procedure, and it would be helpful to monitor tissue growth. For example, devices have been proposed for placement in a urethra of a patient to retain patency of the urethra in patients with benign prostate hyperplasia (BPH). Work in relation to the present disclosure suggests that such implants may be less than ideally effective, and it could be helpful to monitor growth of tissue near the implant. Also, with cancer patients, it would be helpful to know if tissue is growing in a treated area such as site of a resected tumor.

Prior approaches to male fertility and sexual function can be less than ideal in at least some respects. Although medications have been proposed, these can have a less than ideal efficacy in at least some instances.

Some patients may have urinary urgency, and the prior approaches to treating urinary urgency can be less than ideal in at least some instances. For example, in some instances some patients may feel a desire to urinate when their bladder contains relatively little urine.

WO 2015/200723 A1 relates to polymers, systems and methods for using and monitoring polymers for use in medical polymers, implants, and procedures.

WO 2009/151876 A2 relates to coded heterofunctional sutures and methods.

In light of the above, it would be desirable to have improved methods and apparatuses for monitoring and treating patients.

### SUMMARY

In some embodiments, the present disclosure is directed to an implant with one or more sensors to measure one or more fluids or tissues of the patient. The sensor is configured to measure tissue growth in the patient. The one or more sensors may comprise one or more of an impedance sensor, an optical sensor, a light source and a detector to measure back scattered light, a pressure sensor, a strain sensor, a pH sensor, a camera, a marker sensor, a biomarker sensor, a protein sensor, a hormone sensor or an enzyme sensor. The implant is configured for placement in the prostate. In embodiments which are not part of the invention, the implant can be configured for placement in a brain, a heart, a lung, an intestine, an eye, a skin, a kidney, a liver, a pancreas, a stomach, a uterus, an ovary, a testicle, a bladder, an ear, a nose, a mouth, a soft tissue, bone marrow, adipose tissue, a muscle, glandular tissue, mucosal tissue, a spinal tissue, a nerve tissue, cartilage, hard biological tissue, teeth, bone, a body lumen, a passage, a sinus, a ureter, a colon, an esophagus, a lung passage, a blood vessel, or a throat. The sensor may comprise a mechanical sensor configured to measure growth of tissue, such as BPH or a tumor.

In some embodiments, an implant comprising a plurality of electrodes is configured to stimulate one or more of nerve tissue or muscle tissue in order to improve male sexual function.

In some embodiments, an implant comprises one or more stimulation electrodes and one or more sensors to measure a response to stimulation from the one or more electrodes.

### BRIEF DESCRIPTION OF THE DRAWINGS

A better understanding of the features, advantages and principles of the present disclosure will be obtained by reference to the following detailed description that sets forth illustrative embodiments, and the accompanying drawings of which:
FIG. 1 shows a prostate and an implant coupled to the prostate, in accordance with some embodiments;
FIG. 2 shows the implant of FIG. 1, in accordance with some embodiments;
FIG. 3 shows an implant comprising a plurality of sensors, in accordance with some embodiments;
FIG. 4 shows an implant as shown in FIG. 3 placed in a delivery device, in accordance with some embodiments; and
FIG. 5 shows a prostate and locations of the prostate suitable for placement of one or more stimulation electrodes, in accordance with some embodiments.

### DETAILED DESCRIPTION

The following detailed description and provides a better understanding of the features and advantages of the inventions described in the present disclosure in accordance with the embodiments disclosed herein. Although the detailed description includes many specific embodiments, these are provided by way of example only and should not be construed as limiting the scope of the inventions disclosed herein.

While embodiments of the present disclosure are specifically directed to treatment of the prostate, certain aspects of the disclosure, which are not part of the invention, may also be used to treat and modify other organs and tissue such as brain, heart, lungs, intestines, eyes, skin, kidney, liver, pancreas, stomach, uterus, ovaries, testicles, bladder, ear, nose, mouth, soft tissues such as bone marrow, adipose tissue, muscle, glandular and mucosal tissue, spinal and nerve tissue, cartilage, hard biological tissues such as teeth, bone, as well as body lumens and passages such as the sinuses, ureter, colon, esophagus, lung passages, blood vessels, and throat. The devices disclosed herein may be inserted through an existing body lumen, or inserted through an opening created in body tissue.

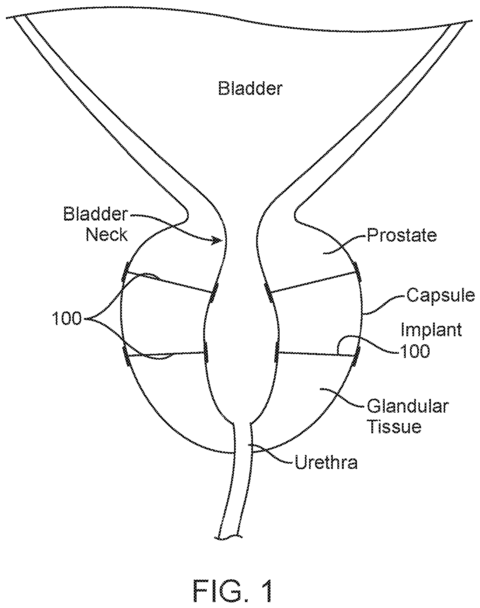

FIG. 1 shows a prostate and an implant 100 coupled to the prostate. The prostate comprises a capsule and glandular tissue. A urethra extends along an interior of the prostate from a bladder neck to a distal opening.

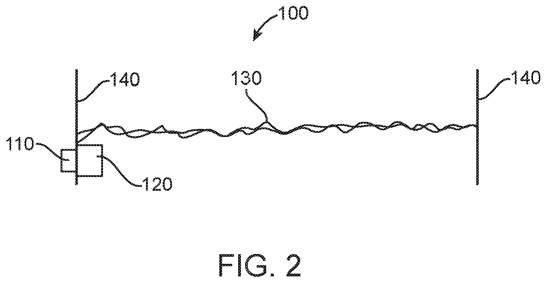

FIG. 2 shows the implant of FIG. 1, in accordance with some embodiments. The implant 100 comprises a sensor 110, circuitry 120, and a support 130. The implant may comprise additional components such as a loop antenna, a power source and a biocompatible coating, for example. Circuitry 120 is coupled to the sensor 110 to transmit signals from the sensor to an exterior of the patient. The circuitry may comprise a processor and wireless transmission circuitry. The sensor may comprise one or more of an impedance sensor, an optical sensor, a light source and a detector to measure back scattered light, a pressure sensor, a strain sensor, a pH sensor, a camera, or a sensor to measures tissue growth in the patient. The sensor may comprise the pH sensor, in which the pH sensor is configured to measure a pH of urine in a urethra of a prostate of the patient. Any suitable number of implants can be used, and the implants can be placed at a plurality of locations, for example.

The sensor can be configured to detect one or more of a marker, a biomarker, a protein, a hormone, or an enzyme.

The implant may comprise a delivery mechanism at one or more locations of the implant, such as on the support, or anchor and combinations thereof. The drug delivery mechanism may comprise a sustained release mechanism. The drug delivery mechanism may comprise a drug eluting mechanism on the support or other location of the implant. The therapeutic agent may comprise one or more of a drug, a hormone, an enzyme, or any other material capable of providing therapy to the patient.

According to the invention, the support comprises an expandable, retractable or tensioning support, and optionally combinations thereof. The support can be coupled to one or more sensors as described herein. Not according to the invention, the implant can be placed in any body lumen such as a blood vessel or a lung, or an airway, and the support and anchors configured to retain the implant in the lumen.

The support may comprise an expandable support or a tensioning support, and combinations thereof. The support comprises one or more structures to engage tissue, and may comprise one or more anchors. The anchors can be configured to engage a capsule of the prostate, or a urethra, for example. In some embodiments, the support comprises a tensioning suture configured to draw the walls of the urethra toward the prostate. The sensor can be configured to measure strain or force along the support, which can be related to tissue grow, such as a cancer. In some embodiments, a plurality of implants can be used to measure a shape change of an organ as described herein.

In some embodiments, the implant comprises an agent delivery mechanism coupled to the support. The agent may comprise a therapeutic agent, such as one or more of a drug, a hormone or an enzyme, etc. The delivery mechanism can be configured for rapid release of the therapeutic agent, e.g. 90% of dose delivered in less than 24 hours. Alternatively or in combination, the delivery mechanism can be configured for sustained release over an extended time, e.g. no more than 10% delivered within 24 hours.

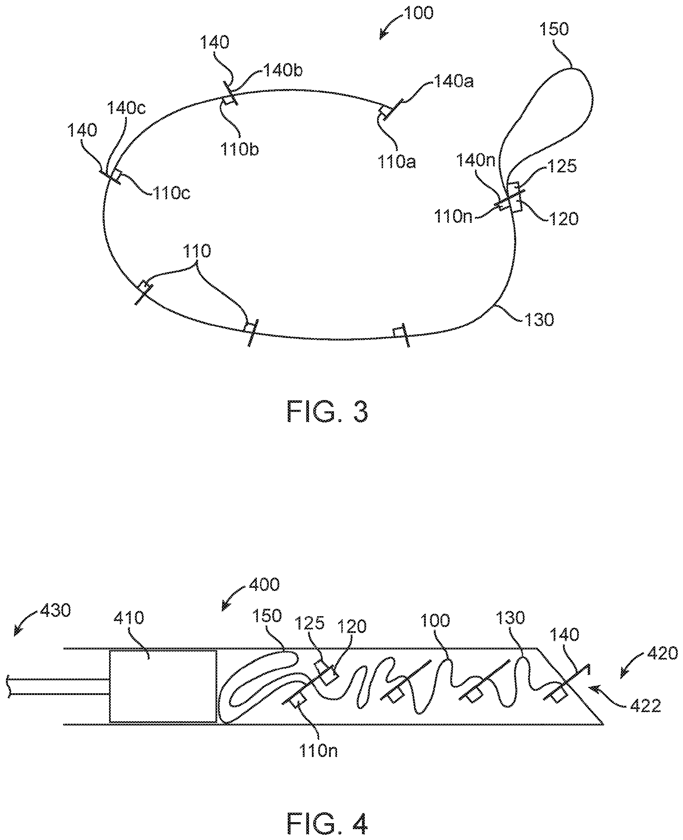

FIG. 3 shows an implant 100 comprising a plurality of sensors 110. The implant 100 comprises a plurality of sensors 110, circuitry 120, a power source 125, a support 130, a plurality of optional anchors 140 and an antenna 150 such as a charging loop antenna. The plurality of sensors 110 may comprise a first sensor 110a, a second sensor 110b, a third sensor 110c up and additional sensors including an Nth sensor 110h, for example. The plurality of sensors 110 is coupled to the processor and the transmitter to process the signals from the sensors and to transmit data derived from the signals to the exterior of the patient. The plurality of anchors 140 may comprise a first anchor 140a, a second anchor 140b, a third anchor 140c and an Nth anchor 140n, for example.

The plurality of sensors 110 and the plurality of anchors 140 are coupled to the support 130 and the circuitry 120. In some embodiments, a biocompatible coating material encloses components of the implant 100, such as the transmitter, the power source, and the processor. The coating material may comprise any suitable coating material such as parylene or silicone, for example.

The support 130 may comprise any suitable material to couple the plurality of sensors 110 to the circuitry 130, and may comprise one or more electrical lines to couple each of the sensors to the circuitry 120 and the power source 125. The support may comprise a flexible material, e.g. a flex PCB, which allows the sensors to be placed at any desired location. In some embodiments, each of the plurality of sensors is coupled to an anchor, such that each sensor can be placed at a desired target location and held in place at the target location with the anchor. The anchors can be configured in many ways, and each may comprise a sharp tip with a barb, for example. The distance between pairs of sensors 110 along support 130 can be set sufficiently large in order to allow the anchors and sensors to be freely positioned at desired locations. The sensors can be placed at locations to establish a spatial relationship profile, which can provide a differential signal, for example. In some embodiments, the distances between pairs of sensors along the support 130 are set, e.g. at predetermined distances, which can facilitate placement in some instances, for example where the sensors are set at target distances. The set distances can be used to set up an intended distance between sensors, such as a spatial relationship profile.

The circuitry 120 comprises circuitry suitable for processing signals from the sensors for transmission with the transmitter, and may comprise any suitable circuitry, such as one or more of analog signal processing circuitry, digital signal processing circuitry, gate logic, gate array logic, programmable array logic, or a digital processor. The circuitry 120 may comprise transmission circuitry such as wireless communication circuitry. The circuitry 120 may comprise a power source to store power such as a super capacitor or a battery for example.

The antenna 150 can be configured in many ways. In some embodiments, antenna 150 comprises an inductive loop coil configured to couple to another coil outside the patient in order to charge the power source and power components of the implant. In some embodiments, circuitry 120 comprises wireless transmission circuitry, and the circuitry is configured to transmit patient sensor data outside the patient with the coil. In some embodiments, the antenna 150 comprises an insulated conductive lead positionable to allow induction charging of the power source of the implant. In some embodiments, the lead is sized and shaped for placement in a urethra so as to extend along the urethra. The lead may be sized and shaped for placement in a urethra so as to extend along the urethra to an exterior of the patent through an opening of the urethra near an end of a penis of a patient, for example. Not according to the invention, the lead may be sized and shaped for subdermal placement under a skin of an abdomen or an appendage.

It should be appreciated that although antenna 150 is shown located on an end of implant 100 in FIG. 3, other configurations can be suitably employed in accordance with the present disclosure. In some embodiments, the support 130 comprises at least a portion of antenna 150 so as to define a loop. In some embodiments antenna 150 extends between a first end of support 130 and a second end of support 150 so as to define a loop with support 130 and antenna 150. The first end of support 130 may comprise a location of the support proximate first sensor 110a and first anchor 140a, and the second end of support 130 may comprise a second end of support 130 proximate Nth sensor 110h and Nth anchor 140n. Alternatively or in combination, the support 130 may comprise a loop comprising one or more coils, with the plurality of sensors and the plurality of anchors located thereon.

The sensors can be configured in many ways and may comprise a component of the support extending between the plurality of anchors. In some embodiments, the plurality of sensors comprises one or more of a plurality of strain gauges along the support or a plurality of piezoelectric sensors along the support. Alternatively or in combination, the sensors may comprise force sensors to measure forces or deflections between the anchors.

In some embodiments, the sensor comprises a pressure sensor configured to measure urinary pressure. The pressure sensor can be located along the urethra or within the bladder, and combinations thereof, in order to measure the pressure of the urine. The pressure of the urine can be compared with the patient's urinary urgency in order to assess the validity of the patient's perceived urinary urgency.

The circuitry on the implant can be configured to store patient data from the sensors, and may comprise a clock to time stamp data received from the sensors for comparison with other patient data. The time stamped sensor data can then be exported to an external device.

In some embodiments, data from the one or more sensors can be received and stored and compared with other data from the user. For example, the implant can be configured to communicate with a mobile computing device such as a smart phone, and the mobile computing device configured to receive and store data from the implant 100. In some embodiments, the mobile computing device comprises application software ("app") loaded onto the mobile computing device, in which the app is configured to communicate with implant 100. In some embodiments, an antenna such as a coil is coupled to the mobile computing device to receive data from the implant and to transmit instructions to the circuitry of the implant.

In some embodiments, the implant 100 comprises a combination of one or more sensors and one or more electrodes. In some embodiments, the circuitry of the implant is configured to stimulate the tissue with the electrodes and to measure the pressure in response to the stimulation. This measurement of pressure in response to electrical stimulation can be helpful to measure male sexual function such as ejaculatory function as described herein, and to measure pressure in response to electrical stimulation of tissues related to urinary urgency and continency such the pressure in response to stimulation of one or more of bladder tissues, prostate tissues, and sphincter muscles, for example.

The implant 100 may comprise one or more stimulation electrodes, e.g. a plurality of simulation electrodes, either alternatively to the sensors 110 or in combination with the sensors 110 as described herein. The stimulation electrodes can be located at any suitable location along support 130 for placement in tissue. The stimulation electrodes can be located in proximity to the anchors 140. In some embodiments, the anchors 140 comprise stimulation electrodes. The stimulation electrodes can be coupled to the circuitry 120 and driven with a drive current to stimulate tissue. In some embodiments, the anchors 140 comprise stainless steel needles electrically coupled to circuitry 120 with electrical lines, e.g. traces, extending along support 130. The circuitry 120 is configured with instructions to stimulate the tissue with the electrodes.

The electrodes can be sized and located on the support in many ways. In some embodiments, the plurality of electrodes is configured for placement on a capsule muscularis of the patient, and each of the plurality of electrodes is positioned on support 130 to allow the electrodes to be appropriately placed. In some embodiments, the electrodes are placed on the capsule muscularis at strategic locations corresponding to improved stimulation. The circuitry comprises a processor coupled to the power source and the plurality of electrodes. The processor is configured to stimulate the capsule muscularis with the electrodes to generate an ejaculation.

The circuitry and processor can be configured in many ways to stimulate the tissue of the capsule muscularis. In some embodiments, the processor is configured to stimulate the capsule muscularis with one or more of a frequency or a cadence. In some embodiments, the processor is configured to stimulate the capsule muscularis with one or more of a varying intensity of electrical stimulation.

In some embodiments, the implant is configured to be activated by the user, for example to provide one or more of erectile function, ejaculation, or continence. While the implant can be activated in many ways, in some embodiments, the implant is activated in response to one or more of an under-skin button, a remote activation, voice activation, Wi-Fi activation, Bluetooth activation, radio activation, mobile device activation, or inductive coupling activation. In some embodiments, the circuitry is configured to respond to a first input to activate electrodes and a second input to deactivate the electrodes, for example in response to one or more user inputs to a touch screen of a mobile device.

FIG. 4 shows the implant 100 placed in a delivery device 400. The delivery device 400 may comprise a cartridge comprising the implant 100. The delivery device 400 may comprise a tubular structure with an interior for storing the implant 100. The delivery device 400 may comprise a distal end 420 for delivering implant 100. In some embodiments, the implant 100 is released from an opening 422 in the distal end 420 to the tissue. In some embodiments, the delivery device 400 comprises a plunger 410 for pushing the implant 100 through the opening 422 on the distal end. The plunger 410 may be coupled to a proximal portion 430 of the delivery device 400, which can be operated by a user to deploy the implant 100.

The implants, sensors and electrodes as disclosed herein can be placed at one or more of many suitable locations. In some embodiments the electrodes are configured for placement at a plurality of locations in a plurality of tissues to generate a synergistic effect. The electrodes can be placed to promote one or more aspects of male sexual function and fertility, such as one or more of ejaculation, erection, or mixing of seminal fluids and prostatic secretions. The electrodes can be placed to promote forward (antegrade) ejaculation, release of sperm, sperm mixing with seminal fluids and prostatic secretions within the prostatic fossa prior to ejaculation. Examples of suitable placement locations include one or more of the bladder neck, the external sphincter, the neurovascular bundle, or the capsule muscularis, which is the prostatic muscle near the capsule. The contraction of the prostatic muscle can help to pressurize semen in the prostatic fossa, for example. This pressurization can be measured with a pressure sensor placed in a lumen of the urethra as described herein, for example.

Referring again to FIG. 4, the delivery device 400 can be used to deliver the implant 100 comprising a plurality of electrodes to a plurality of specific locations. The electrodes can be delivered sequentially to the plurality of locations. Each of the electrodes can be configured to stimulate adjacent tissue, so as to stimulate specific nerves or muscles. Examples of suitable electrode placement locations include one or more of the capsule muscularis, the bladder neck sphincter, the external sphincter, the prostate capsule, the neurovascular bundle, or the subtunical vein plexus, which provides tumescence for the treatment of erectile dysfunction.

In some embodiments, the stimulation of tissue is tested with each of the plurality of electrodes prior to anchoring the electrode in the tissue. Alternatively or in combination, the electrodes can be adjusted when placed to calibrate the electrodes and the amount of stimulation current in order to achieve a desired outcome, such as one or more of erectile function, ejaculation, or continence.

Work in relation to the present disclosure suggests that the type of tissue in proximity to the electrode can influence the appropriate amount of voltage and/or current to use to stimulate the tissue. In some embodiments, placement near neurological tissue can provide stimulation with decreased amounts of current as compared with electrodes placed in muscular or capsular tissue.

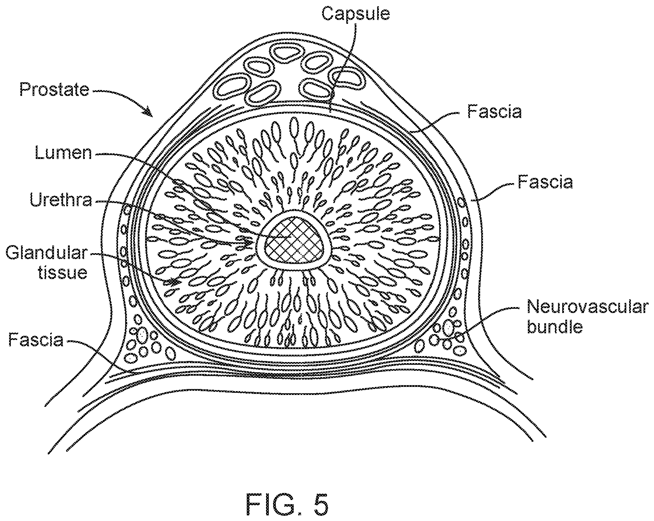

FIG. 5 shows a prostate and suitable locations for placement of one or more stimulation electrodes. The prostate comprises a capsule and a urethra. The urethra comprises a lumen along which urine or semen may flow. Glandular tissue of the prostate is located between the urethra and the capsule. A neurovascular bundle of the of nerve fibers and blood vessels can extend along the exterior of the prostate. One or more layers of fascia can extend around the prostate. The tissues of the prostate can be stimulated in many ways and at many locations. In some embodiments, an electrode is placed directly in a neurological tissue to stimulate one or more nerves, such as nerves of the neurovascular bundle. The electrode of the implant can be advanced from the urethra so as to extend through the capsule and simulate the neurovascular bundle. In some embodiments, the implant is anchored to the capsule or a layer of fascia extending around the capsule, for example. In some embodiments, the electrode is configured to stimulate the neurovascular bundle with the one or more electrodes only extending partially into the neurovascular bundle. In some embodiments, the one or more electrodes extends through the neurovascular bundle in order to stimulate the neurovascular bundle. Alternatively or in combination, one or more electrodes can be placed so as to stimulate the neurovascular bundle without extending through the neurovascular bundle. For example, in some embodiments the one or more electrodes is placed in an adjacent tissue such as the capsule or fascia, so as not to extend into the neurovascular bundle, and the current path from the one or more electrodes is configured to extend into the neurovascular bundle so as to stimulate the neurons and nerves of the neurovascular bundle. Although reference is made to placing the one or more electrodes with access from the urethra, in some embodiments, the tissues of the prostate such as the neurovascular bundle are accessed from outside the prostate, for example with abdominal access or perineal access.

The electrodes can be placed similarly with respect to other tissues as described herein, such as one or more of the capsule muscularis, the bladder neck sphincter, the external sphincter, the prostate capsule, or the subtunical vein plexus. The electrodes of the implant can be placed at one or more of these locations, and the support as described herein can extend between a plurality of electrodes at these locations.

In some embodiments, the current provided by the circuitry is adjusted in situ after the electrodes have been placed. The amount of current, waveform, and other parameters to each of the plurality of electrodes can be adjusted to determine appropriate stimulation parameters to provide a desired outcome as described herein. In some embodiments, the circuitry is configured to adjust the amount of current to each electrode independently of other electrodes, such that the appropriate amount of stimulation to each electrode can be used. In some embodiments, low amounts of stimulating current are provided followed by increasingly greater amounts. In some embodiments, the patient is alert and can provide feedback to the medical personnel tuning the circuitry for the appropriate amount of energy. In some embodiments, the current to each of the plurality of electrodes is adjusted to provide one or more of erectile function, ejaculation, or continence. In some embodiments, one or more sensors are employed to measure the efficacy of the stimulus. For example, a balloon can be placed in the prostate to measure contraction of the prostate. Alternatively or in combination, one or more structures comprising sensors can be placed on the penis to measure tumescence in response to the pattern of electrical stimulation.

The implants comprising electrodes as disclosed herein can be configured in many ways to stimulate tissue. In some embodiments, the electrodes and circuitry are configured to generate a contraction of the prostate muscles, produce an erection, or produce an ejaculation, and combinations thereof. In some embodiments, the electrodes and sensors are configured to assess urinary urgency. For example, some people can feel a compelling urge to urinate, even though the need to do this may not be real. The strain gauges as disclosed herein can be used to measure tensioning of the prostate tissue and to determine if the strain of the prostate is consistent with a perceived need to urinate. Alternatively or in combination, the implant may comprise a pressure sensor as described herein to determine whether there is sufficient pressure for the user to appropriately perceive a sense of urgency to urinate.

Although reference is made to treatment of the prostate in males, the presently disclosed methods and apparatus are well suited to treatment of females in other tissues and organs besides the prostate.

Although reference is made to stimulation of tissue with electrodes, other stimuli can be used, such as one or more of light, sound, vibrations, or chemical stimulation. These other stimuli can be used alternatively or in combination with electrodes as described herein to stimulate tissue. The one or more of light, sound, vibrations, or chemical stimulation can be used at the locations described herein in the context of electrodes in order to treat one or more conditions of the patient.

Embodiments of the present disclosure have been shown and described as set forth herein and are provided by way of example only. One of ordinary skill in the art will recognize numerous adaptations, changes, variations and substitutions without departing from the scope of the present disclosure. Several alternatives and combinations of the embodiments disclosed herein may be utilized without departing from the scope of the present disclosure and the inventions disclosed herein. Therefore, the scope of the presently disclosed inventions shall be defined solely by the scope of the appended claims.

## Claims

1. An implant (100) for placement in a patient, comprising:
a support (130);
a transmitter on the support (130);
a sensor (110) on the support (130), the sensor (110) being configured to measure tissue growth in the patient; and
circuitry (120) coupled to the sensor (110) and the transmitter to transmit signals from the sensor (110) to an exterior of the patient;
wherein the support (130) comprises an expandable support (130) sized and shaped for placement in a prostate of the patient to maintain patency of the urethra along the prostate, and wherein the sensor (110) is coupled to the expandable support (130) to measure deflection of the expandable support (130) and wherein deflection of the support (130) is related to tissue growth within the prostate.

2. The implant (100) of claim 1, wherein the sensor (110) comprises one or more of an impedance sensor, an optical sensor, a light source and a detector to measure back scattered light, a pressure sensor, a strain sensor, a pH sensor, a camera, a marker sensor, a biomarker sensor, a protein sensor, a hormone sensor or an enzyme sensor and optionally wherein the pH sensor is configured to measure a pH of urine in a urethra of a prostate of the patient.

3. The implant (100) of claim 1, wherein the support (130) comprises a tensioning support (130) sized and shaped for placement in a prostate of the patient to maintain patency of the urethra along the prostate and, optionally, wherein the sensor (110) is configured to measure deflection of the support (130).

4. The implant (100) of claim 1, wherein the support (130) comprises a barb to engage a capsule of a prostate.

5. The implant (100) of claim 1, further comprising an agent delivery mechanism coupled to the support (130) and optionally wherein the agent comprises a therapeutic agent and optionally wherein the therapeutic agent comprises one or more of a drug, a hormone or an enzyme.

6. The implant (100) of claim 5, wherein the delivery mechanism is configured for rapid release and optionally wherein the delivery mechanism is configured to release a therapeutic dosage over a time of less than a day.

7. The implant (100) of claim 5, wherein the delivery mechanism is configured for sustained release over an extended time and optionally wherein the extended time comprises at least a week.

8. The implant (100) of claim 1, further comprising:
a power source (125) coupled to the circuitry (120) and the transmitter to process signals from the sensors (110) and to transmit the signals;
a biocompatible material enclosing the transmitter, the power source (125), and the circuitry (120); wherein the transmitter comprises wireless communication circuitry; and wherein the sensor (110) comprises a plurality of sensors (110a, 110b, 100c) coupled to the circuitry (120).

9. The implant (100) of claim 1, further comprising an insulated conductive lead positionable to allow induction charging of a power source (125) of the implant (100).

10. The implant (100) of claim 9, wherein the lead is sized and shaped for placement in a urethra so as to extend along the urethra optionally to an exterior of the patient through an opening of the urethra near an end of a penis of the patient.

11. The implant (100) of claim 1, wherein the sensor (110) comprises a plurality of sensors (110a, 110b, 110c) coupled to the circuitry (120) with an electrically conductive line, the line extending between the plurality of sensors (110a, 110b, 110c) to allow the plurality of sensors (110a, 110b, 110c) to be placed at a plurality of locations.

## Patentansprüche

1. Implantat (100) zur Platzierung in einem Patienten, umfassend:
einen Träger (130);
einen Sender auf dem Träger (130);
einen Sensor (110) auf dem Träger (130), wobei der Sensor (110) konfiguriert ist, um Gewebewachstum in dem Patienten zu messen; und
eine Schaltung (120), die mit dem Sensor (110) und dem Sender gekoppelt ist, um Signale von dem Sensor (110) zu einem Äußeren des Patienten zu übertragen;
wobei der Träger (130) einen expandierbaren Träger (130) umfasst, der zur Platzierung in einer Prostata des Patienten bemessen und geformt ist, um Durchgängigkeit der Harnröhre entlang der Prostata aufrechtzuerhalten, und wobei der Sensor (110) mit dem expandierbaren Träger (130) gekoppelt ist, um Auslenkung des expandierbaren Trägers (130) zu messen, und wobei Auslenkung des Trägers (130) mit Gewebewachstum innerhalb der Prostata in Beziehung steht.

2. Implantat (100) nach Anspruch 1, wobei der Sensor (110) eines oder mehrere von einem Impedanzsensor, einem optischen Sensor, einer Lichtquelle und einem Detektor zum Messen von rückgestreutem Licht, einem Drucksensor, einem Dehnungssensor, einem pH-Sensor, einer Kamera, einem Markersensor, einem Biomarkersensor, einem Proteinsensor, einem Hormonsensor oder einem Enzymsensor umfasst, und wobei optional der pH-Sensor konfiguriert ist, um einen pH-Wert von Urin in einer Harnröhre einer Prostata des Patienten zu messen.

3. Implantat (100) nach Anspruch 1, wobei der Träger (130) einen Spannträger (130) umfasst, der zur Platzierung in einer Prostata des Patienten bemessen und geformt ist, um Durchgängigkeit der Harnröhre entlang der Prostata aufrechtzuerhalten, und wobei optional der Sensor (110) konfiguriert ist, um Auslenkung des Trägers (130) zu messen.

4. Implantat (100) nach Anspruch 1, wobei der Träger (130) einen Widerhaken umfasst, um mit einer Kapsel einer Prostata in Eingriff zu gelangen.

5. Implantat (100) nach Anspruch 1, ferner umfassend einen Wirkstoffabgabemechanismus, der mit dem Träger (130) gekoppelt ist, und wobei optional der Wirkstoff einen therapeutischen Wirkstoff umfasst, und wobei optional der therapeutische Wirkstoff eines oder mehrere von einem Arzneimittel, einem Hormon oder einem Enzym umfasst.

6. Implantat (100) nach Anspruch 5, wobei der Abgabemechanismus zur schnellen Freisetzung konfiguriert ist, und wobei optional der Abgabemechanismus konfiguriert ist, um eine therapeutische Dosis über eine Zeit von weniger als einem Tag freizusetzen.

7. Implantat (100) nach Anspruch 5, wobei der Abgabemechanismus zur anhaltenden Freisetzung über eine verlängerte Zeit konfiguriert ist, und wobei optional die verlängerte Zeit mindestens eine Woche umfasst.

8. Implantat (100) nach Anspruch 1, ferner umfassend:
eine Leistungsquelle (125), die mit der Schaltung (120) und dem Sender gekoppelt ist, um Signale von den Sensoren (110) zu verarbeiten und die Signale zu übertragen;
ein biokompatibles Material, das den Sender, die Leistungsquelle (125) und die Schaltung (120) umschließt; wobei der Sender eine Schaltung zur drahtlosen Kommunikation umfasst; und wobei der Sensor (110) eine Vielzahl von Sensoren (110a, 110b, 100c) umfasst, die mit der Schaltung (120) gekoppelt sind.

9. Implantat (100) nach Anspruch 1, ferner umfassend eine isolierte leitfähige Leitung, die positionierbar ist, um Induktionsladen einer Leistungsquelle (125) des Implantats (100) zu ermöglichen.

10. Implantat (100) nach Anspruch 9, wobei die Leitung zur Platzierung in einer Harnröhre bemessen und geformt ist, um sich entlang der Harnröhre optional zu einem Äußeren des Patienten durch eine Öffnung der Harnröhre nahe einem Ende eines Penis des Patienten zu erstrecken.

11. Implantat (100) nach Anspruch 1, wobei der Sensor (110) eine Vielzahl von Sensoren (110a, 110b, 110c) umfasst, die mit der Schaltung (120) mit einer elektrisch leitfähigen Leitung gekoppelt sind, wobei sich die Leitung zwischen der Vielzahl von Sensoren (110a, 110b, 110c) erstreckt, um ein Platzieren der Vielzahl von Sensoren (110a, 110b, 110c) an einer Vielzahl von Stellen zu ermöglichen.

## Revendications

1. Implant (100) destiné à être placé dans un patient, comprenant :
un support (130) ;
un émetteur sur le support (130) ;
un capteur (110) sur le support (130), le capteur (110) étant configuré pour mesurer la croissance tissulaire chez le patient ; et
un circuit (120) couplé au capteur (110) et à l'émetteur pour transmettre des signaux du capteur (110) à l'extérieur du patient ;
le support (130) comprenant un support expansible (130) dimensionné et formé pour être placé dans la prostate du patient afin de maintenir la perméabilité de l'urètre le long de la prostate, et le capteur (110) étant couplé au support expansible (130) pour mesurer la déviation du support expansible (130), et la déviation du support (130) étant liée à la croissance tissulaire dans la prostate.

2. Implant (100) selon la revendication 1, le capteur (110) comprenant un ou plusieurs des éléments parmi : un capteur d'impédance, un capteur optique, une source de lumière et un détecteur pour mesurer la lumière rétrodiffusée, un capteur de pression, un capteur de contrainte, un capteur de pH, une caméra, un capteur de marqueur, un capteur de biomarqueur, un capteur de protéine, un capteur d'hormone ou un capteur d'enzyme, et, éventuellement, le capteur de pH étant configuré pour mesurer le pH de l'urine dans l'urètre de la prostate du patient.

3. Implant (100) selon la revendication 1, le support (130) comprenant un support de tension (130) dimensionné et formé pour être placé dans la prostate du patient afin de maintenir la perméabilité de l'urètre le long de la prostate et, éventuellement, le capteur (110) étant configuré pour mesurer la déviation du support (130).

4. Implant (100) selon la revendication 1, le support (130) comprenant une barbe pour s'engager dans une capsule d'une prostate.

5. Implant (100) selon la revendication 1, comprenant en outre un mécanisme d'administration d'agent couplé au support (130) et, éventuellement, l'agent comprenant un agent thérapeutique et, éventuellement, l'agent thérapeutique comprenant un ou plusieurs éléments parmi un médicament, une hormone ou une enzyme.

6. Implant (100) selon la revendication 5, le mécanisme d'administration étant configuré pour une libération rapide et, éventuellement, le mécanisme d'administration étant configuré pour libérer une dose thérapeutique sur une période inférieure à une journée.

7. Implant (100) selon la revendication 5, le mécanisme d'administration étant configuré pour une libération soutenue sur une période prolongée et, éventuellement, la période prolongée comprenant au moins une semaine.

8. Implant (100) selon la revendication 1, comprenant en outre :
une source d'alimentation (125) couplée au circuit (120) et à l'émetteur pour traiter les signaux provenant des capteurs (110) et pour transmettre les signaux ;
un matériau biocompatible renfermant l'émetteur, la source d'alimentation (125) et le circuit (120) ; l'émetteur comprenant un circuit de communication sans fil ; et le capteur (110) comprenant une pluralité de capteurs (110a, 110b, 110c) couplés au circuit (120).

9. Implant (100) selon la revendication 1, comprenant en outre un fil conducteur isolé pouvant être positionné pour permettre la charge par induction d'une source d'alimentation (125) de l'implant (100).

10. Implant (100) selon la revendication 9, le fil étant dimensionné et formé pour être placé dans l'urètre de manière à s'étendre le long de l'urètre, éventuellement jusqu'à l'extérieur du patient, à travers une ouverture de l'urètre près d'une extrémité du pénis du patient.

11. Implant (100) selon la revendication 1, le capteur (110) comprenant une pluralité de capteurs (110a, 110b, 110c) couplés au circuit (120) par une ligne électriquement conductrice, la ligne s'étendant entre la pluralité de capteurs (110a, 110b, 110c) pour permettre à la pluralité de capteurs (110a, 110b, 110c) d'être placés à une pluralité d'emplacements.
